Europäisches Patentamt

(19) European Patent Office

Office européen des brevets

(11) EP 0 767 646 B1

(12) EUROPEAN PATENT SPECIFICATION

(45) Date of publication and mention
of the grant of the patent:
08.09.1999 Bulletin 1999/36

(21) Application number: 95922306.6

(22) Date of filing: 12.06.1995

(51) Int. Cl.$^6$: **A61F 13/15**, A61F 13/52

(86) International application number:
PCT/US95/07435

(87) International publication number:
WO 96/00549 (11.01.1996 Gazette 1996/03)

(54) **FLUID PERVIOUS WEB EXHIBITING A SURFACE ENERGY GRADIENT**

FLÜSSIGKEITSDURCHLÄSSIGES GEWEBE MIT OBERFLÄCHENENERGIEGRADIENTEN

BANDE PERMEABLE AU FLUIDE PRESENTANT UN GRADIENT D'ENERGIE SUPERFICIELLE

(84) Designated Contracting States:
AT BE CH DE DK ES FR GB GR IE IT LI LU NL PT
SE

(30) Priority: 30.06.1994 US 268404
20.10.1994 US 326571

(43) Date of publication of application:
16.04.1997 Bulletin 1997/16

(73) Proprietor:
THE PROCTER & GAMBLE COMPANY
Cincinnati, Ohio 45202 (US)

(72) Inventors:
• OUELLETTE, William, Robert
Cincinnati, OH 45240 (US)
• LEE, Yann-Per
Fairfield, OH 45014 (US)
• HANEY, Anna, Renee
Cincinnati, OH 45245 (US)
• LANGDON, Frederick, Michael
Kobe 658 (JP)
• BURCHNALL, John, Billings
West Chester, OH 45069 (US)

(74) Representative:
Hirsch, Uwe Thomas et al
Procter & Gamble European Service GmbH,
Sulzbacher Strasse 40-50
65824 Schwalbach am Taunus (DE)

(56) References cited:
EP-A- 0 040 084          EP-A- 0 205 286
EP-A- 0 207 904          EP-A- 0 235 309
EP-A- 0 272 118          EP-A- 0 304 617
EP-A- 0 483 859          EP-A- 0 596 532
WO-A-93/03699            WO-A-94/22408
CA-A- 1 033 903          US-A- 4 077 410

EP 0 767 646 B1

## Description

FIELD OF THE INVENTION

The present invention relates to a fluid pervious web suitable for use as a topsheet on a disposable absorbent article, and more particularly, to such a web exhibiting a surface energy gradient. In particular, the portion of the web which faces and/or contacts the wearer's skin, i.e., the wearer-contacting surface, preferably has a relatively low surface energy while the remaining portions of the web have a relatively high surface energy.

BACKGROUND OF THE INVENTION

It has long been known in the disposable absorbent article art that it is extremely desirable to construct absorptive devices, such as disposable diapers, sanitary napkins, incontinent briefs, bandages, wound dressings, and the like, presenting a dry surface feel to the user to improve wearing comfort and to minimize the development of undesirable skin conditions due to the prolonged exposure to moisture absorbed within the article. Nonwoven type topsheet materials are known from EP 235 309 which addresses the above problem by providing a nonwoven fabric of two layers of different fibre compositions which are entangled with one another.

One viable prior art solution to the aforementioned problem is disclosed in commonly assigned U.S. Pat. No. 4,342,314 issued to Radel et al. on August 3, 1982 and hereby incorporated herein by reference. Radel et al. discloses an absorbent article with a wearer-contacting topsheet comprising a resilient, macroscopically expanded, three-dimensional plastic web exhibiting a combination of fiber-like and plastic properties. The term "fiber-like", as utilized herein to describe the appearance of plastic webs, refers generally to any fine scale pattern of embossments or apertures, random or non-random, reticulated or non-reticulated, which connotes an overall appearance and impression of a woven or nonwoven fibrous web when viewed by the human eye. When describing the elements used to form the web, the term "fiber-like" is utilized herein to describe the appearance or shape of the elements. As utilized herein, the term "macroscopically expanded", when used to describe three-dimensional plastic webs, ribbons and films, refers to webs, ribbons and films which have been caused to conform to the surface of a three-dimensional forming structure so that both surfaces thereof exhibit the three-dimensional pattern of said forming structure, said pattern being readily visible to a normal naked eye having 20/20 vision when the perpendicular distance between the viewer's eye and the plane of the web is about 12 inches. Such macroscopically expanded webs, ribbons and films are typically caused to conform to the surface of said forming structures by embossing, i.e., when the forming structure exhibits a pattern comprised primarily of male projections, by debossing, i.e., when the forming structure exhibits a pattern comprised primarily of female capillary networks, or by extrusion of a resinous melt directly onto the surface of a forming structure of either type. By way of contrast, the term "planar" when utilized herein to describe plastic webs, ribbons and films, refers to the overall condition of the web, ribbon and film when viewed by the naked eye on a macroscopic scale. In a preferred embodiment the macroscopically expanded, three-dimensional plastic web topsheet disclosed in Radel et al. exhibits a fine scale three-dimensional microstructure comprising a regulated continuum of capillary networks, of steadily decreasing size, originating in and extending from one surface of the web and terminating in the form of apertures in the opposite surface thereof to promote rapid fluid transport in the direction of decreasing capillary size. As utilized herein, the term "microstructure" refers to a structure of such fine scale that its precise detail is readily perceived by the human eye only upon magnification by a microscope or means well-known in the art. The web's fiber-like appearance is comprised of a continuum of fiber-like elements, the opposed ends of each of the fiber-like elements being interconnected to at least one other of the fiber-like elements.

A typical capillary network in the Radel et al. structure comprises an uppermost capillary opening formed by a multiplicity of primary fiber-like elements interconnected to one another in the uppermost plane of the web. The uppermost opening may, if desired, be further subdivided into smaller capillary openings by secondary and tertiary fiber-like elements at planes located below the wearer-contacting surface of the web.

Each of the fiber-like elements exhibits a substantially uniform U-shaped cross-section along its length. In the case of a primary fiber-like element, its cross-section comprises a base portion located in the wearer-contacting plane and a sidewall portion joined to each edge of the base portion and extending generally in the direction of the absorbent pad-contacting surface of the web. The sidewall portions which intersect one another are joined to one another intermediate the wearer-contacting surface and the absorbent pad-contacting surface of the web, thereby forming a capillary network interconnecting the opposed surfaces of the web. The secondary and tertiary fiber-like elements, when employed are generally similar, but originate in planes below the wearer-contacting surface of the web. Similarly EP 596 532 discloses a laminated film material for use as a body contacting layer in absorbent articles to provide improved surface dryness which comprises a thermoplastic film material bonded in a spaced apart bonding pattern to a nonwoven. Also EP 304 617 describes a sheet of material to envelop the absorbent of a sanitary article which comprises a hydrophobic film having slanted wall openings to prevent rewet. EP 272 118 describes topsheet materials having improved fluid passage and decreased re-wet which have a hydrophilic surface provided by a rubber like material coating.

One drawback associated with the use of topsheets comprised of plastic is that despite their superior fluid handling characteristics some users are very reluctant to place a topsheet which they readily perceive as plastic by virtue of its glossy appearance in contact with their skin. To reduce the gloss on the web's visible surface, i.e., that portion of the web which is visible from directly overhead, it has been learned that the inclusion of a microscopic pattern of surface aberrations which are not discernible when the perpendicular distance between the viewer's eye and the plane of the web is about 12 inches is highly effective. Commonly assigned US Pat. No. 4463045 issued to Ahr et al. on July 31, 1984 and hereby incorporated herein by reference defines the relevant criteria which must be satisfied so that the three-dimensionally expanded web will exhibit a substantially non-glossy visible surface.

A topsheet of the type generally disclosed in Radel et al. having surface aberrations according to Ahr et al., exhibits a fiber-like appearance and tactile impression as well as a non-glossy visible surface. In addition, it is highly effective in promoting rapid fluid transfer from the first or wearer-contacting surface to the second or absorbent pad-contacting surface of the topsheet. Topsheets of the latter type have enjoyed widespread commercial access on catamenial pads due to their clean and dry appearance in use when contrasted to conventional nonwoven fibrous topsheets.

Nonetheless, it will be readily appreciated that even further improvements in the clean and dry appearance and consumer tactile properties in use of topsheets of any material type are highly desirable in disposable absorbent products.

## SUMMARY OF THE INVENTION

The present invention pertains, in a preferred embodiment, to a fluid-pervious web comprising a first or wearer-contacting surface and a second or garment-facing surface. The web is particularly well suited for use as a topsheet on a disposable absorbent article. The first and said second surfaces are separated from one another by an intermediate portion. The first surface of the web exhibits a surface energy less than the surface energy of the intermediate portion. In a preferred embodiment, the web is an apertured formed film. The intermediate portion of the web preferably exhibits a surface energy less than the surface energy of the second surface of the web.

The first surface of the web may include a plurality of zones exhibiting different surface energies. The zones may extend in a direction substantially parallel to the longitudinal axis of the web.

In another embodiment, the web of the present invention is a coated, apertured, nonwoven web comprising a first surface and a second surface. The first and said second surfaces are separated from one another by an intermediate portion. The first surface of the web has a fluid impervious coating thereon. A plurality of apertures extend from the coating on the first surface to the second surface of the web to render the web fluid pervious. The coating exhibits a surface energy less than the surface energy of the intermediate portion.

In another embodiment, the present invention also pertains to an absorbent article including a topsheet, a backsheet secured to said topsheet, and an absorbent core positioned between said topsheet and said backsheet. The absorbent article has a longitudinal axis, a transverse axis perpendicular to the longitudinal axis and a "Z" axis extending perpendicular to both the longitudinal axis and the transverse axis. The topsheet comprises a first surface and a second surface. The first and second surfaces are separated from one another by an intermediate portion. The first surface of the web exhibits a surface energy less than the surface energy of the intermediate portion. The absorbent article preferably includes an acquisition layer positioned between the topsheet and the absorbent core.

## BRIEF DESCRIPTION OF THE DRAWINGS

While the specification concludes with claims particularly pointing out and distinctly claiming the present invention, it is believed that the present invention will be better understood from the following description in conjunction with the accompanying drawings, in which like reference numbers identify identical elements and wherein:

FIG. 1 is a top plan view of a sanitary napkin with portions of the sanitary napkin cut-away to more clearly show the construction of the sanitary napkin;

FIG. 2 is a cross-sectional view of the sanitary napkin of FIG. 1 taken along section line 2-2;

FIG. 3 is an enlarged, partially segmented, perspective illustration of a prior art plastic web of the type generally disclosed in U.S. Pat. No. 4,342,314;

FIG. 4 is an enlarged, partially segmented perspective illustration of a preferred plastic web of the present invention having a sub energy gradient;

FIG. 5 is an enlarged, cross-sectional view of one web construction according to the present invention;

FIG. 6 is an enlarged, cross-sectional view of an alternative web construction according to the present invention;

FIG. 7 is an enlarged cross-sectional view of a drop of liquid on a solid surface, where angle θ, illustrates the contact angle of the liquid with the solid surface;

FIG. 8 is a top plan view of the topsheet portion of a sanitary napkin embodiment of the present invention;

FIG. 9 is a top plan view of the topsheet portion of another sanitary napkin embodiment of the present invention;

FIG. 10 is an enlarged, partially segmented, perspective illustration of another preferred embodiment of a nonwoven web of the present invention;

FIG. 11 is an enlarged, cross-sectional view similar to FIG. 5 of the nonwoven web of FIG. 10.

FIG. 12 is a greatly enlarged simplified schematic cross-sectional illustration of a "planar" polymeric web suitable for use in the present invention illustrating an overall fine scale pattern of surface abberations depicting the presence of a microaperture at the peak of each surface aberration;

FIG. 13 is a schematic illustration depicting the response of the web of FIG. 12 and the papillary ridges of the skin on the wearer's finger to lateral movement between the observer's finger and the uppermost surface of the polymeric web;

FIG. 14 is a greatly enlarged simplified perspective illustration of a segment of a microapertured "planar" web of the type generally illustrated in cross-section in FIG. 12;

FIG. 15 is a greatly enlarged simplified schematic cross-sectional illustration of a "macroscopically expanded" three-dimensional web formed using a "planar" web similar to that of FIG. 12 as a starting material; and

FIG. 16 is a view generally similar to that of FIG. 15, but showing an alternative embodiment of a "macroscopically-expanded", macroscopically apertured three-dimensional polymeric web formed using a "planar" web similar to that of FIG. 12 as a starting material.

FIG. 17 is a view generally similar to that of FIG. 16, but showing in greater detail the application of a coating to the web of FIG. 16.

## DETAILED DESCRIPTION OF THE PRESENT INVENTION

As used herein, the term "absorbent article" refers to devices which absorb and contain body exudates, and more specifically refers to devices which are placed against or in proximity to the body of the wearer to absorb and contain the various exudates discharged from the body. The term "absorbent article" is intended to include diapers, catamenial pads, sanitary napkins, pantiliners, incontinent pads, and the like, as well as bandages and wound dressings. The term "disposable" is used herein to describe absorbent articles which are not intended to be laundered or otherwise restored or reused as an absorbent article (i.e., they are intended to be discarded after a single use, and, preferably, to be recycled, composted or otherwise disposed of in an environmentally compatible manner). A "unitary" absorbent article refers to absorbent articles which are formed as separate parts united together to form a coordinated entity so that they do not require separate manipulative parts like a separate holder and pad.

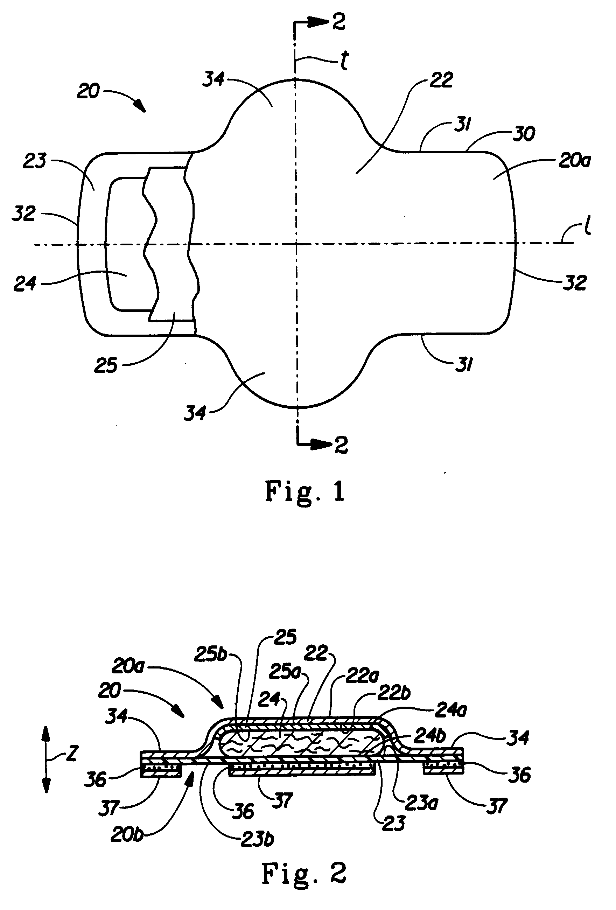

A preferred embodiment of a unitary disposable absorbent article is the catamenial pad, sanitary napkin 20, shown in FIG. 1. As used herein, the term "sanitary napkin" refers to an absorbent article which is worn by females adjacent to the pudendal region, generally external to the urogenital region, and which is intended to absorb and contain menstrual fluids and other vaginal discharges from the wearer's body (e.g., blood, menses, and urine). Interlabial devices which reside partially within and partially external to the wearer's vestibule are also within the scope of this invention. As used herein, the term "pudendal" refers to the externally visible female genitalia. It should be understood, however, that the present invention is also applicable to other feminine hygiene or catamenial pads such as pantiliners, or other absorbent articles such as diapers, incontinent pads, and the like.

A sanitary napkin 20 has two surfaces, a first surface 20a sometimes referred to as a wearer-contacting or facing surface, a body-contacting or facing surface or "body surface" and a second surface 20b, sometimes referred to as a garment-facing or contacting surface, or "garment surface". The sanitary napkin 20 is shown in FIG. 1 as viewed from its first surface 20a. The first surface 20a is intended to be worn adjacent to the body of the wearer. The second surface 20b of the sanitary napkin 20 (shown in FIG. 2) is on the opposite side and is intended to be placed adjacent to the wearer's undergarment when the sanitary napkin 20 is worn.

The sanitary napkin 20 has two centerlines, a longitudinal centerline "l" and a transverse centerline "t". The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" as used herein, are interchangeable and refer to a line, axis or direction which lies within the plane of the sanitary napkin 20 that it generally perpendicular to the longitudinal direction. FIG. 1 also shows that the sanitary napkin 20 has a periphery 30 which is defined by the outer edges of the sanitary napkin 20 in which the longitudinal edges (or "side edges") are designated 31 and the end edges (or "ends") are designated 32.

FIG. 1 is top plan view of the sanitary napkin 20 of the present invention in its flat-out state with portions of the sanitary napkin being cut-away to more clearly show the construction of the sanitary napkin 20 and with the portion of the sanitary napkin 20 which faces or contacts the wearer 20a oriented towards the viewer. As shown in FIG. 1, the sanitary napkin 20 preferably comprises a liquid pervious topsheet 22, a liquid impervious backsheet 23 joined with the topsheet 22, an absorbent core 24 positioned between the topsheet 22 and the backsheet 23, and a secondary topsheet or acquisition layer 25 positioned between the topsheet 22 and the absorbent core 24.

The sanitary napkin 20 preferably includes optional side flaps or "wings" 34 that are folded around the crotch portion of the wearer's panty. The side flaps 34 can serve a number of purposes, including, but not limited to protecting the wearer's panty from soiling and keeping the sanitary napkin secured to the wearer's panty.

FIG. 2 is a cross-sectional view of the sanitary napkin 20 taken along section line 2-2 of FIG. 1. As can be seen in FIG. 2, the sanitary napkin 20 preferably includes an adhesive fastening means 36 for attaching the sanitary napkin 20 to the undergarment of the wearer. Removable release liners 37 cover the adhesive fastening means 36 to keep the adhesive from sticking to a surface other than the crotch portion of the undergarment prior to use.

The topsheet 22 has a first surface 22a and a second surface 22b. The topsheets second surface 22b is positioned adjacent to and is preferably secured to the first surface 25a of the acquisition layer 25 to promote fluid transport from the topsheet to the acquisition layer. The second surface 25b of the acquisition layer 25 is positioned adjacent to and is preferably secured to the first surface 24a of the absorbent core 24 to promote fluid transport from the acquisition layer to the absorbent core. The second surface 24b of the absorbent core 24 is positioned adjacent to and is preferably secured to the first surface 23a of the backsheet 23.

In addition to having a longitudinal direction and a transverse direction, the sanitary napkin 20 also has a "Z" direction or axis, which is the direction preceding down through the topsheet 22 and into whatever fluid storage core 24 that may be provided. The objective is to provide a continuous path between the topsheet 22 and the underlying layer or layers of the absorbent article herein, such that fluid is eventually drawn in the "Z" direction and away from the topsheet of the article and then to its ultimate storage layer.

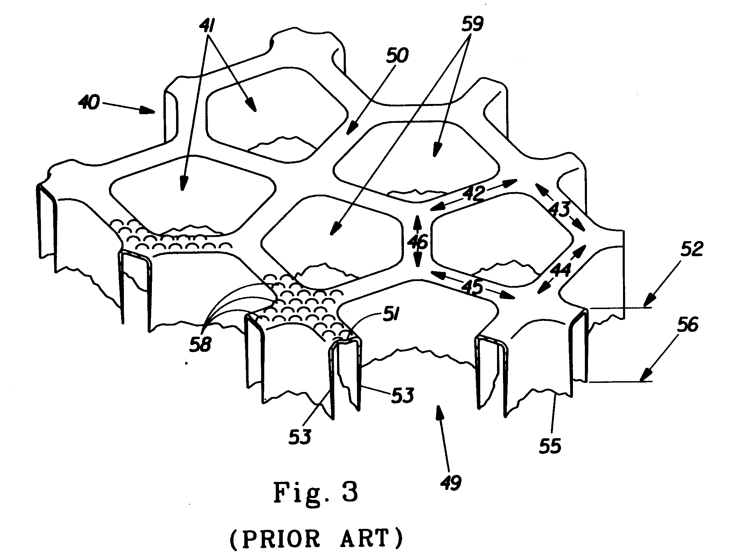

FIG. 3 is an enlarged partially segmented, perspective illustration of a prior art three-dimensional, fiber-like fluid pervious plastic web 40 which has been found highly suitable for use as a topsheet in disposable absorbent articles, such as a sanitary napkin topsheet 22 in a sanitary napkin 20 of the type generally illustrated in FIGS. 1 and 2. The prior art web 40 is generally in accordance with the teachings of commonly assigned U.S. Pat. No. 4,342,314 issued to Radel et al. on August 3, 1982 and hereby incorporated herein by reference. The fluid pervious plastic web 40 exhibits a multiplicity of apertures, e.g., apertures 41, which are formed by a multiplicity of intersecting fiber-like elements, e.g., elements 42, 43, 44, 45, and 46, interconnected to one another in the first or wearer-contacting surface 50 of the web. Each fiber-like element comprises a base portion, e.g., base portion 51 located in plane 52. Each base portion has a sidewall portion, e.g., sidewall portions 53, attached to each edge thereof. The sidewall portions extend generally in the direction of the second surface 55 of the web. The intersecting sidewall portions of the fiber-like elements are interconnected to one another intermediate the first and second surfaces of the web, and terminate substantially concurrently with one another in the plane 56 of the second surface 55.

In a particularly preferred embodiment, the base portion 51, includes a microscopic pattern of surface aberrations 58, generally in accordance with the teachings of commonly assigned U.S. Pat. No. 4,463,045 issued to Ahr et al. on July 31, 1984 and hereby incorporated herein by reference. The microscopic pattern of surface aberrations 58 provides a substantially non-glossy visible surface when the web is struck by incident light rays.

In a particularly preferred embodiment, the interconnected sidewall portions 53 terminate substantially concurrently with one another in the plane 56 of the second surface 55 to form apertures 49 in the second surface 55 of the web. The capillary networks 59 formed by the interconnected sidewall portions allows for the free transfer of fluid from the first or wearer-contacting surface 50 of the web directly to the second surface 55 of the web without lateral transmission of fluid between adjacent capillary networks.

Typically, the prior art web 40 when used as a topsheet on an absorbent article is treated with a surfactant to render the topsheet hydrophilic. The exposed surfaces of the base portions 51 and the sidewall portions 53 are treated with a surfactant such that they will both be rendered substantially hydrophilic, thereby diminishing the likelihood that body fluids will flow off the topsheet rather than being drawn through the topsheet and thereby absorbed by the absorbent core. Suitable methods of applying surfactants are described in U.S. Pat. Nos. 4,950,254 and 5,009,563 both issued to Osborn.

Despite the effective functioning of the surfactant treated prior art fluid-pervious web 40 in topsheet applications for disposable absorbent articles such as sanitary napkins, there are certain drawbacks associated with this particular topsheet. For example, treating the entire exposed surface of the topsheet with a surfactant creates a very wettable surface which when placed into contact with the wearer's skin, may cause the topsheet to stick to the wearer's skin. Thereby, creating a hot, sweaty, and/or sticky sensation for the user which may be viewed as undesirable by some users.

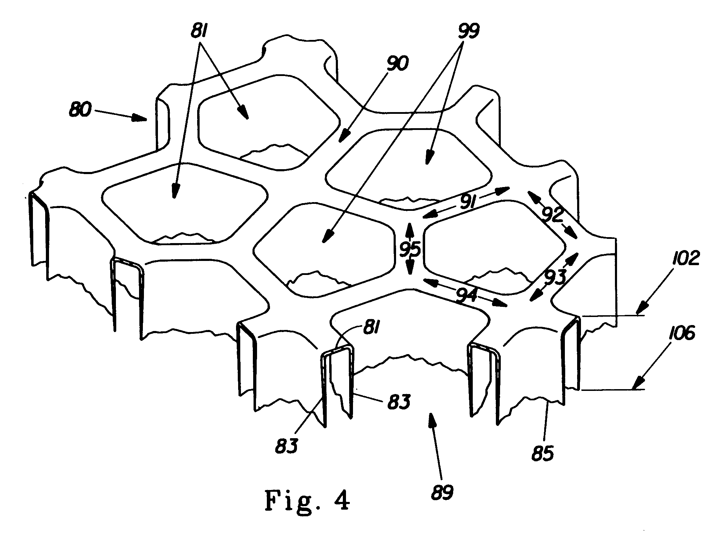

FIG. 4 is an enlarged partially segmented, perspective illustration of a three-dimensional, fiber-like, fluid-pervious formed-film web embodiment of the present invention, generally indicated as 80. The geometrical configuration of the fluid pervious web 80 is generally similar to that of prior art web 40 illustrated in FIG. 3 and is generally in accordance with the teachings of U.S. Pat. No. 4,342,314 issued to Radel et al. on August 3, 1982.. Other suitable formed films are described in U.S. Pat. No. 3,929,135, issued to Thompson on December 30, 1975; U.S. Pat. No. 4,324,246 issued to Mullane, et al. on April 13, 1982; U.S. Pat. No. 4,463, 045 issued to Ahr, et al. on July 31, 1984; and U.S. Pat. No. 5,006,394 issued to Baird on April 9, 1991. Each of these patents are incorporated herein by reference. The web 80 is preferably made from a thermoplastic film. Examples of suitable materials for use as the web 80 include but are not limited to polyolefins such as polyethylenes, including linear low density polyethylene, low density polyethylene, ultra low density polyethylene, high density polyethylene, and polypropylene; metallocene catalyst-based polymers; nylon (polyamide); cellulose esters; poly (methyl methacrylate); polystyrene; poly (vinyl chloride); polyester; polyurethane; compatible polymers; biodegradable polymers; and blends, laminates and/or combinations thereof. Films made from such materials may be plasticized with suitable additives known in the art. Other additives may be added to achieve the desired physical characteristics.

The fluid pervious plastic web 80 exhibits a multiplicity of apertures, e.g., apertures 81, which are formed by a multiplicity of intersecting fiber-like elements, e.g., elements 91, 92, 93, 94, and 95, interconnected to one another in the first or wearer-contacting surface 90 of the web. Each fiber-like element comprises a base portion, e.g., base portion 81 located in plane 102. Each base portion has a sidewall portion e.g., sidewall portions 83, attached to each edge thereof. The sidewall or intermediate portions 83 extend generally in the direction of the second surface 85 of the web. The intersecting sidewall portions of the fiber-like elements are interconnected to one another intermediate the first and second surfaces of the web, and terminate substantially concurrently with one another in the plane 106 of the second surface 85.

In a particularly preferred embodiment, the interconnected sidewall or intermediate portions 83 terminate substantially concurrently with one another in the plane 106 of the second surface 85 to form apertures 89 in the second surface 85 of the web. The capillary networks 99 formed by the interconnected sidewall or intermediate portions 83 allows for the free transfer of fluid from the first or wearer-contacting surface 90 of the web directly to the second surface 85 of the web without lateral transmission of fluid between adjacent capillary networks.

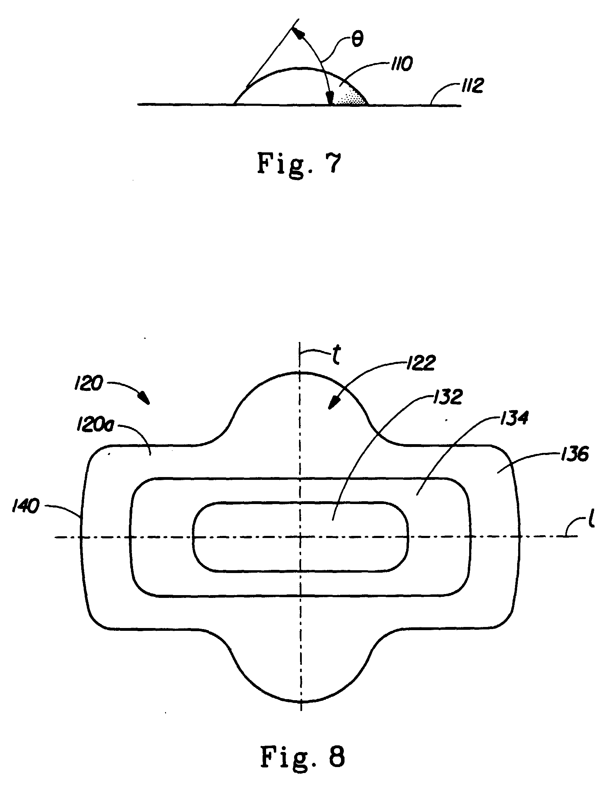

In a particularly preferred embodiment, the first or wearer-contacting surface 90 of web 80 is relatively non-wettable compared to the relatively wettable intermediate or sidewall portions 83. A useful parameter of wettability is the contact angle that a drop of liquid (gas-liquid interface) makes with the solid surface (gas-solid surface). Typically, a drop of liquid 110 placed on a solid surface 112 makes a contact angle, $\theta$, with the solid surface, as seen in FIG. 7. As the wettability of the solid by the liquid increases the contact angle, $\theta$, decreases. As the wettability of the solid by the liquid decreases the contact angle, $\theta$, increases. The liquid-solid contact angle may be determined from techniques known in the art.

The contact angle depends on surface inhomogeneities (chemical and physical, such as roughness), contamination, chemical/physical treatment of the solid surface, as well as the nature of the liquid and its contamination. The surface energy of the solid also influences the contact angle. As the surface energy of the solid decreases, the contact angle increases. As the surface energy of the solid increases, the contact angle decreases.

The energy required to separate a liquid from a solid surface, (e.g., a film or fiber), is expressed by the equation:

$$W_a = \gamma(1 + \cos\theta)$$

where

$W_a$ is the work of adhesion measured in $erg/_{cm}2$,
$\gamma$ is the surface tension of the liquid measured in dyne/cm, and
$\theta$ is the liquid-solid contact angle measured in degrees.

With a given liquid, the work of adhesion increases with the cosine of the liquid-solid contact angle (reaching a maximum where the contact angle is zero).

While the first or wearer-contacting surface 90 of web 80 has a relatively low surface energy and a relatively low work of adhesion for a given fluid, e.g., water, the sidewall or intermediate portions 83 of the web 80 preferably have a relatively high surface energy and a relatively high work of adhesion for a given fluid. Water is used as a reference liquid throughout and is not meant to be limiting. To the contrary, the concepts regarding work of adhesion with respect to water can easily be applied to other fluids such as blood, menses and urine. Since the intermediate portions 83 of the web 80 has a relatively higher surface energy as compared to the first surface 90, the intermediate portions 83 are more wettable than the first surface 90.

The second surface 85 of the web 80 preferably has a higher surface energy and a higher work of adhesion for water than that of the first surface 90. The second surface's 85 surface energy and work of adhesion for water may be the same as that of the intermediate portion 83. In a preferred embodiment, the second surface's 85 surface energy and work of adhesion for water are relatively higher than that of the intermediate portion 83.

By having a web with a surface energy gradient, that is, a relatively low surface energy which contacts the wearer's skin, i.e., the first surface 90, and a relatively high surface energy portion not in contact with the wearer's skin, i.e., the sidewall or intermediate portions 83; the web 80 is capable of moving a drop of liquid from the portion of the web exhibiting the relatively lower surface energy to the portion of the web exhibiting the relatively higher surface energy. The motion of the drop of liquid is induced by the contact angle differential between the lower surface energy portion and the higher surface energy portion which results in an imbalance in surface tension force acting on the solid-liquid contact plane. It is the surface energy gradient, which enhances the fluid handling properties of the web 80 of the present invention and which makes the web well suited for use as a topsheet on an absorbent article, such as topsheet 22 on absorbent article 20 illustrated in FIG 1.

In addition to the enhanced fluid handling properties, by having the relatively lower surface energy portion of the web 80 in contact the wearer's skin, the adhesion between the skin and the web is reduced by decreasing the capillary force generated by occlusive body fluids located between the first surface of the web and the wearer's skin. By reducing the adhesion between the wearer's skin and the web, the sensation or impression of stickiness associated with adhesion to a plastic web topsheet is reduced.

The potential for rewet is also reduced by having a topsheet with a surface energy gradient according to the aforementioned description. As fluid attempts to rewet or be squeezed out of the pad, i.e., squeezed from the absorbent core towards the first surface of the topsheet, it is resisted by the first surface of the topsheet which has a relatively low surface energy to repel the fluid as it attempts to make its way out of the pad through the openings in the topsheet.

Furthermore, fluid is able to enter the topsheet more quickly due to the driving forces of the surface energy gradient of the topsheet. Fluid is moved in the "Z" direction toward the second surface of the topsheet via the surface energy gradient from the relatively lower surface energy first surface to the relatively higher surface energy sidewall portions of the topsheet toward the absorbent core.

Preferably, the first surface 90 of the web 80 has a work of adhesion for water in the range of about 0 $erg/_{cm}2$ to about 150 $erg/_{cm}2$, more preferably in the range of about 0 $erg/_{cm}2$ to about 100 $erg/_{cm}2$, and most preferably in the range of about 0 $erg/_{cm}2$ to about 75 $erg/_{cm}2$. Preferably, the intermediate portion 83 of the web 80 has a work of adhesion for water in the range of about 0 $erg/_{cm}2$ to about 150 $erg/_{cm}2$, more preferably in the range of about 25 $erg/_{cm}2$ to about 150 $erg/_{cm}2$, and most preferably in the rage of about 50 $erg/_{cm}2$ to about 150 $erg/_{cm}2$. Preferably, the second surface 85 of the web 80 has a work of adhesion for water in the range of about 0 $erg/_{cm}2$ to about 150

$erg/_{cm}2$, more preferably in the range of about 25 $erg/_{cm}2$ to about 150 $erg/_{cm}2$, and most preferably in the range of about 50 $erg/_{cm}2$ to about 150 $erg/_{cm}2$.

Preferably, the difference in the work of adhesion for water between the first surface and the intermediate portion of the web is in the range of about 5 $erg/_{cm}2$ to about 145 $erg/_{cm}2$, more preferably in the range of about 25 $erg/_{cm}2$ to about 145 $erg/_{cm}2$, and most preferably in the range of about 50 $erg/_{cm}2$ to about 145 $erg/_{cm}2$.

The surface energy gradient is preferably continuous as it extends from the first surface 90 of the web into and through the intermediate portion 83 of the web. Alternatively, the surface energy gradient may be discrete or stepped as it extends from the first surface 90 of the web into and through the intermediate portion 83 of the web.

To manufacture the web 80 of the present invention having a surface energy gradient, a sheet of polyethylene is extruded onto a drum where it is vacuum formed into an apertured formed film and then subjected to a corona discharge treatment generally in accordance with the teachings of U.S. Pat. Nos. 4,351,784 issued to Thomas et al. on Sep. 28, 1982; 4,456,570 issued to Thomas et al. on Jun. 26, 1984; and 4,535,020 issued to Thomas et al. on Aug. 13, 1985, each of these patents being incorporated herein by reference. The polyethylene may, if desired, have a surfactant incorporated into the resin. A coating having a relatively low surface energy is then applied to the first surface of the apertured formed film and is preferably cured. A suitable coating is a silicone release coating from Dow Corning of Midland, Michigan available as Syl-Off 7677 (90%) / 7048 (10% crosslinker). The surface energy of the silicone release coating on the first surface of the apertured formed film is less than the surface energy of the polyethylene intermediate portions which may have been subjected to the corona discharge treatment.

Suitable surfactants include, for example, ethoxylated esters such as Pegosperse® 200-ML, manufactured by Glyco Chemical, Inc. of Greenwich, Connecticut, glucose amides, tri-block copolymers of ethylene oxide and propylene oxide such as Pluronic® P103, manufactured by BASF, and copolymers of silicone and ethylene glycol such as DC190, manufactured by Dow Corning of Midland, Michigan.

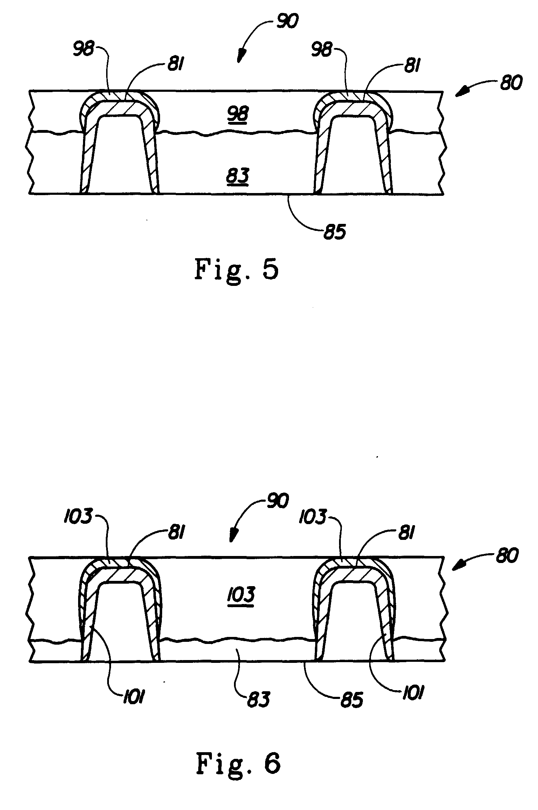

Figure 5 is a cross-sectional illustration more clearly depicting the orientation of the silicone coating 98 on the first surface of the formed film of Figure 4.

Alternatively, the intermediate portion of a corona discharge- and silicone-treated web 80 may be dip coated with a wetting agent such that the intermediate portions 83 have a relatively higher surface energy than the corona discharge- and silicone-treated first surface of the web.

Another preferred method for converting a ribbon of polyethylene film, which may optionally have a surfactant mixed therein, into an apertured formed film is by applying a high pressure fluid jet comprised of water or the like against one surface of the film, preferably while applying a vacuum adjacent the opposite surface of the film. Such methods are described in greater detail in commonly assigned U.S. Pat Nos. 4,609,518 issued to Curro et al. on Sept. 2, 1986; 4,629,643 issued to Curro et al. on Dec. 16, 1986; 4,637,819 issued to Ouellette et al. on Jan. 20, 1987; 4,681,793 issued to Linman et al. on July 21, 1987; 4,695,422 issued to Curro et al. on Sept. 22, 1987; 4,778,644 issued to Curro et al. on Oct. 18, 1988; 4,839,216 issued to Curro et al. on June 13, 1989; and 4,846,821 issued to Lyons et al. on July 11, 1989, each of said patents being incorporated herein by reference. The apertured formed film is then subjected to a corona discharge treatment. A silicone release coating, may then be coated or printed onto the first surface of the apertured formed film and is preferably cured. The intermediate portion of the apertured, formed film web may be dip coated with a wetting agent such that the intermediate portions 83 have a relatively higher surface energy than does the corona discharge- and silicone-treated first surface of the web. The surface energy of the silicone-treated first surface is less than the surface energy of the intermediate portions.

Figure 6 depicts another method of forming a formed film having a surface energy gradient as in the present invention is to utilize a multilayer film in a forming process such as the one described above. The first layer 99 of the film which will constitute the first surface of the web is formed of a first material, while the second layer 101 of the film which will constitute the second surface of the web is formed of a second material. The second material preferably exhibits a greater ductility and a higher surface energy than the second material. During the aperturing process, the first layer of the film fractures first while the second layer of the film stretches to a greater extent to form the lower portion of the web. The first surface of the finished web is thus comprised of the first material, while the intermediate and lower portions of the finished web are comprised of the second material exposed following the fracture of the first material. The finished web thus exhibits a surface energy gradient from the first surface to the intermediate portion to the second surface without the additional steps of treating the first, intermediate, and/or second surfaces with additives or coatings. The first and second layers herein described may be separated from one another by one or more intervening layers in a multilayer film having 3 or more layers which may optionally participate in the surface energy gradient of the film by having intermediate surface energy characteristics.

Referring now to FIG. 8 there is shown another preferred embodiment of a sanitary napkin 120 of the present invention. The sanitary napkin 120 is shown in FIG. 8 as viewed from its first or wearer-contacting surface 120a. The sanitary napkin 120 includes a liquid pervious topsheet 122, a liquid impervious backsheet (not shown), joined with the topsheet 122, an absorbent core (not shown), positioned between the topsheet 122 and the backsheet, and an acquisition layer (not shown) positioned between the topsheet 122 and the absorbent core.

The topsheet 122 includes a plurality of regions and/or zones. Preferably the topsheet 122 includes a first central region 132, a second region 134 adjacent to and contiguous with the first region 132, and a third region 136 adjacent to and contiguous with the second region 134. Preferably, the first surface of the topsheet 122 within the first central region 132 has a relatively higher surface energy than that of the topsheet 122 within the adjacent second region 134. Likewise, the first surface of the topsheet 122 within the second region 134 has a relatively higher surface energy than that of the topsheet 122 within the adjacent third region 136. Thus, fluid deposited on the topsheet 122 will be driven from the third region 136 toward the second region 134 and from the second region 134 toward the first region 132. Accordingly, fluid will be directed from the third region 136 towards the first region 132 of the topsheet 122 to help prevent any run-off of fluids over the periphery 140 of the sanitary napkin.

While the first or wearer-contacting surface of the topsheet 122 has a surface energy gradient from region to region, which may be discrete or continuous, the topsheet 122 will also preferably have an additional surface energy gradient between the first surface and the sidewall or intermediate portions of the topsheet 122. The surface energy of the sidewall portions 134 within the respective regions of the topsheet, will be higher than the surface energy of the wearer-contacting surface in the first, second and third regions of the topsheet 122. Thus, the topsheet will also promote the transmission of fluids in the "Z" direction similar to that of web 80 disclosed in FIG. 4.

In some situations it may be desirable to have a surface energy gradient on the first surface of the topsheet 122 which forces fluid from the first region to the second region, and from the second region to the third region. In this embodiment, the first surface of the topsheet 122 within the first region 132 has a relatively lower surface energy than that of the topsheet 122 within the adjacent second region 134. Similarly, the first surface of the topsheet 122 within the second region 134 has a relatively lower surface energy than that of the topsheet 122 within the adjacent third region 136. Thus, fluid deposited on the topsheet 122 will be driven from the first region 132 toward the second region 134, and from the second region 134 toward the third region 136. This type of surface energy gradient may be desirable when trying to fully utilize the absorbent capacity of the underlying absorbent core by spreading bodily fluids across the first surface of the topsheet, the fluids will have a more direct path to the peripheral portions of the underlying absorbent core.

The regions or zones 132, 134, 136 are shown in FIG. 8 as generally being of an oval configuration. However, the regions may be formed in a wide variety of shapes and sizes such as rectangular, elliptical, hourglass, dogbone, asymmetric, triangular, circular, etc.

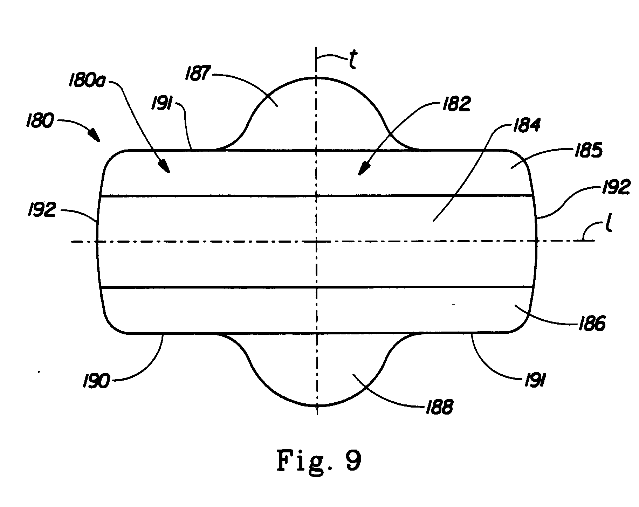

Referring now to FIG. 9 there is shown a sanitary napkin 180 as viewed from its first surface 180a. The sanitary napkin 180 includes elements or components similar to that of sanitary napkin 20 shown in FIGS. 1 and 2 such as a liquid pervious topsheet 182, a liquid impervious backsheet joined with the topsheet 182, an absorbent core positioned between the topsheet 182 and the backsheet, and a secondary topsheet or acquisition layer positioned between the topsheet 182 and the absorbent core. The sanitary napkin 180 has a periphery 190 which is defined by the outer edges of the sanitary napkin 180 in which the longitudinal edges (or "side edges") are designated 191 and the end edges (or "ends") are designated 192.

The topsheet 182 includes a plurality of regions extending generally parallel to the longitudinal axis "l" of the sanitary napkin 180. The topsheet 182 includes a first or central region 184 extending parallel to the longitudinal axis from one end of the sanitary napkin to the other end. Adjacent to the first or central region 184 is a pair of second regions 185, 186 extending essentially parallel to the first region 184. Adjacent the second regions 185, 186, respectively, are a pair of third regions 187, 188. Preferably, the first region has a relatively low surface energy as compared to the second regions 185, 186. Similarly, the second regions 185, 186 have a relatively low surface energy as compared to the third regions 187, 188.

Alternatively, the first region may have a relatively high surface energy as compared to the second regions 185, 186. The second regions 185, 186 may then have a relatively high surface energy as compared to the third regions 187, 188.

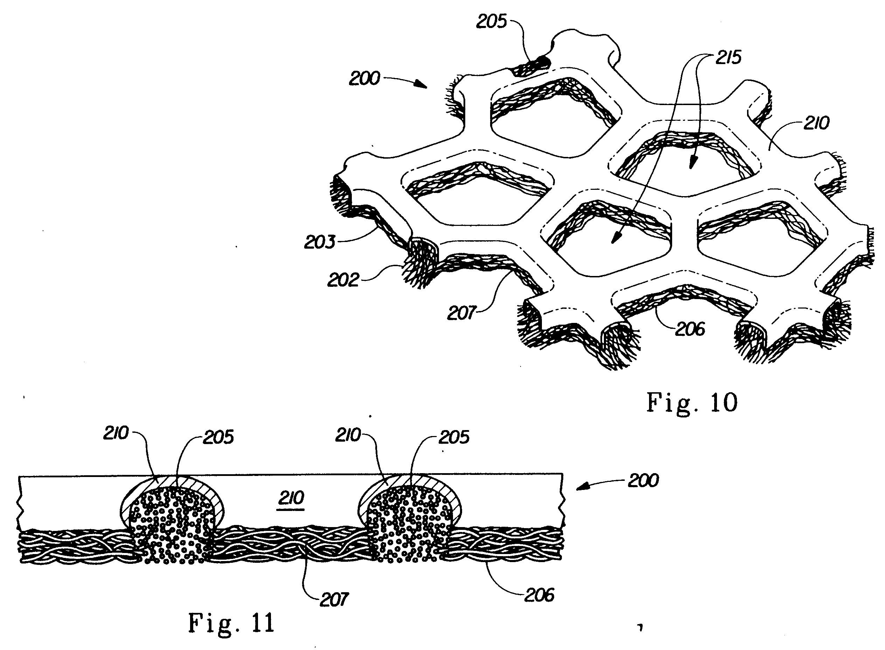

Referring now to FIG. 10 there is shown a cross-sectional view of another embodiment of a fluid pervious web 200 of the present invention. Fluid-pervious web 200 includes a fluid pervious nonwoven web 202 comprised of polypropylene fibers 203. Other suitable fibers include natural fibers such as wood, cotton, or rayon, or synthetic fibers such as polyester or polyethylene, or combinations of natural and synthetic fibers.

The nonwoven web 202 preferably has a first surface 205 and a second surface 206. The first surface 205 is spaced from the second surface 206 by an intermediate portion 207. The first surface 205 preferably has a fluid impervious coating 210 thereon. Preferably, the fluid impervious coating 210 is comprised of curable silicone such as Syl-Off 7677 (90%) / 7048 (10% crosslinker) available from Dow Corning of Midland, Michigan. Other suitable coating materials include but are not limited to fluorinated materials (e.g., Teflon) and Petrolatum. A plurality of apertures 215 extend from the coating 210 to the second surface 206 of the nonwoven web 202.

Preferably, the coating 210 has a relatively low surface energy and a relatively low work of adhesion as compared to the fibers of the nonwoven which have a relatively high surface energy and a relatively high work of adhesion. Accordingly, the coated nonwoven web 200 exhibits a surface energy gradient.

The coating 210 may be applied to the first surface 205 of the nonwoven web 202 by techniques known in the art such as screen printing, gravure printing, spraying, dip coating, etc. The coated nonwoven web 200 may be apertured by techniques known in the art such a needle punching, hydroentangling, etc.

The coating 210 is preferably applied to the first surface of the nonwoven web after the aperturing operation is complete. Alternatively, the coating 210 may be applied to the first surface of the nonwoven web prior to the aperturing operation.

Figure 11 is a cross-sectional illustration more clearly depicting the orientation of the coating 290 on the first surface of the nonwoven web of Figure 10.

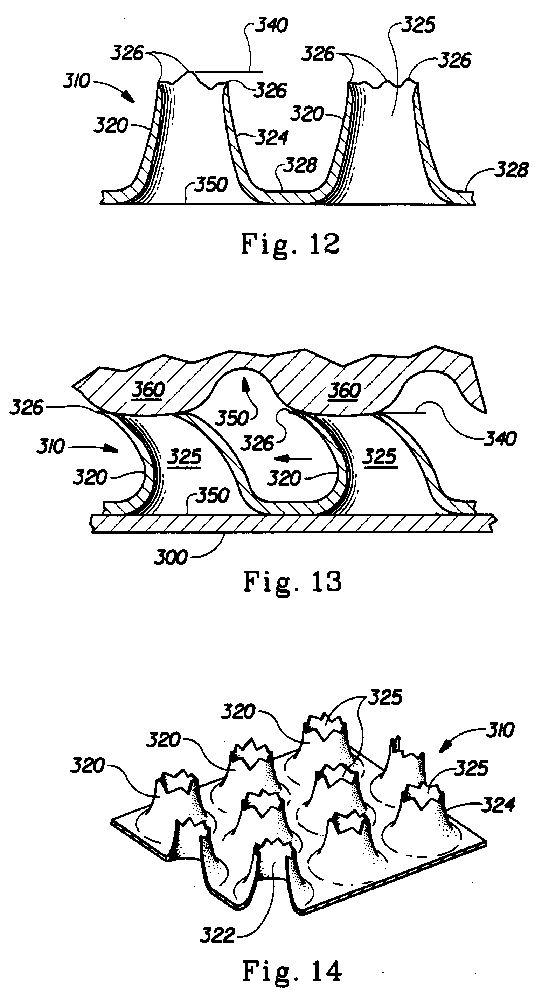

Figures 12-17 illustrate other types of formed films which are suitable for use in accordance with the present invention. These microapertured formed films are described in greater detail in commonly assigned U.S. Patent No. 4,629,643, issued December 16, 1986 to Curro and Linman, and hereby incorporated herein by reference.

FIG. 12 is a greatly enlarged simplified cross-sectional illustration of a microapertured web 310 according to the present invention. It may, if desired, be formed on a woven wire support member. However, rather than relying upon suction to fully conform the web to the surface of the woven wire support member, a high pressure liquid jet is preferably utilized for this purpose (the details of which will be hereinafter set forth). Because the greater driving force applied by the liquid jet, those portions of the web which coincide with the interstices formed by the intersecting filaments in the woven wire support member are ruptured to form tiny apertures, i.e., microaperture 325 at points which coincide substantially with the maximum amplitude of each surface aberration 320.

As can be seen in FIG. 12, rupturing of the surface aberrations 320 at these points results in the formation of a volcano-shaped aperture 325 having relatively thin, irregularly shaped petals 326 about its periphery. As can also be observed from FIG. 12, the outermost extremities of the petals 326 are substantially thin end due to the elongation which occurs just prior to rupture of the film by the high pressure liquid jet. As will be appreciated from an examination of FIG. 12, the overall caliper, i.e., the distance between uppermost plane 340 and lowermost plane 350 of "planar" web 310 is slightly greater than the overall caliper of prior art "planar" webs due to the drawing and thinning which takes place in the end wall of each surface aberration immediately prior to rupture.

The actual size of microaperture 325 is of less importance than its mere existence. The primary benefit of the microaperture on the surface aberration is that it reduces the overall resistance to compression and shear of the surface aberration and destroys its ability to respond as an integral reinforced unit. This provides enhanced tactile properties (i.e., more clothlike) which are particularly desirable for use in absorbent articles which contact particularly sensitive areas of human anatomy.

FIG. 13 is a greatly enlarged simplified illustration showing the response of microapertured web 310 of the present invention supported on a substrate 300 and the papillary ridges 360 on the skin of the observer's finger when the observer's finger 350 is moved laterally across the surface of the microapertured surface aberrations 320. In particular, note that the overall degree of collapse, i.e., the physical distance between uppermost plane 340 and lowermost plane 350, although initially somewhat greater than that of a prior art unapertured "planar" web has been reduced to a level less than that of prior art webs for a comparable loading. This is believed to be due to the elimination of the integral end walls in surface aberrations 320. Because of the existence of microapertures 325 at the center of each of the surface aberrations 320, the overall resistance to collapse of each individual surface aberration 320 is greatly reduced. Furthermore, because of the irregular nature of the petals 326 formed about the periphery of microapertures 325, and the pliable nature of the thinned petals 326, the papillary ridges 360 of the observer's skin are not deflected as much as in prior art webs. It is believed that all of the foregoing factors contribute to the perception that the tactile response of microapertured polymeric "planar" web 310 is substantially softer and silkier than that of prior art webs.

FIG. 14 is a greatly enlarged perspective illustration of a segment of a "planar" microapertured web of the present invention, such as web 310 generally illustrated in FIGS. 12 and 13.

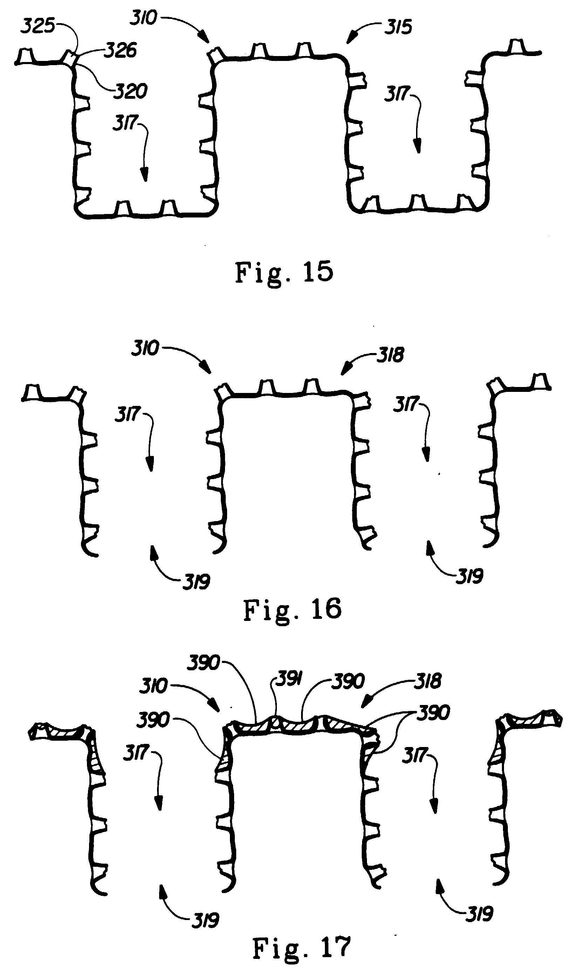

It is of course recognized that microapertured "planar" webs 310 of the present invention may, if desired, be "macroscopically expanded" to form a three-dimensional polymeric web. Exemplary of macroscopically expanded webs are those generally taught by the aforementioned commonly assigned U.S. Pat. No. 4,342,314 to Radel et al., said patent being hereby incorporated herein by reference. Macroscopic expansion is preferably carried out by orienting the microapertured surface aberrations 320 so that microapertures 325 form the skin contacting surface of the macroscopically expanded web 315, as generally shown in FIG. 15. The resilience imparted by macroscopic expansion of the web is superposed on that exhibited by the microapertured surface aberrations 320 of "planar" web 310.

Macroscopic debossments 317 in web 315 may be of any particular shape and size desired by creating a forming structure as generally taught by commonly assigned U.S. pat. No. 4,395,215 issued to Bishop on July 26, 1983 and hereby incorporated herein by reference. The teachings of the Bishop patent are particularly useful in the event macroscopic expansion without macroscopic aperturing of the web is desired. In such case, it is necessary to provide at least one of the lamina in the three-dimensional forming structure with a very fine pattern of apertures sufficient to support the bottom of the macroscopic debossments 317 without permitting macroscopic rupturing thereof during the macroscopic expansion process.

An alternative macroscopically expanded web 318 of the present invention is illustrated in greatly enlarged form in FIG. 16. The web shown in FIG. 16 is generally similar to that shown in FIG. 15, with the exception that the end walls of the debossments 317 have been ruptured to form macroscopic apertures 319. This may be accomplished by using a forming structure of the type disclosed in the aforementioned commonly assigned U.S. Patent No. 4,342,314 to Radel et al., which is incorporated herein by reference.

While Figures 15 and 16 depict microembossments/microapertures oriented in a direction opposite to that of the macroembossments/macroapertures, these illustrations are to be illustrative only and not limiting. That is to say, other relative orientations are contemplated as well, such as those wherein the microembossments/microapertures are oriented in the same direction as the macroembossments/macroapertures.

Figure 17 is a cross-sectional view of one of the macroscopic structures of Figure 16, more clearly depicting the presence of fluid-impervious coating 390 on the upper surface of the structure. As shown in Figure 17, under some circumstances the coating may at least partially obstruct at least some of the microapertures 325 (as indicated at 391). This tends to further reduce the surface energy of the upper surface of the web in comparison with the intermediate portion having unobstructed microapertures. In addition, partial or total obstruction of microapertures on the uppermost portion of the formed film with the coating further reduce the likelihood that fluid will be trapped inside the microapertures, thus enhancing the feeling of dryness experienced by the wearer.

More specific details as to the nature of the processes which may be utilized to manufacture the microapertured, macroscopically expanded and/or apertured formed films depicted in Figures 15 and 16 are set forth in commonly assigned U.S. Patent No. 4,609,518, issued September 2, 1986 to Curro et al., and hereby incorporated herein by reference. Following manufacture of the microapertured formed films depicted in Figures 12-17, the surface energy gradient properties of the present invention are imparted to the formed films in the manner described above with respect to Figures 3 and 4, and they may be incorporated into absorbent articles such as those depicted in Figures 8 and 9. Indeed, the surface energy gradient properties of the present invention are particularly useful in combating the tendency of fluids to accumulate in and around the microstructures present in formed films such as depicted in Figures 15-17. This leads to webs having improved clothlike characteristics without sacrificing apparent consumer dryness.

In addition to the formation processes described above, the surface energy gradients according to the present invention may be applied to film structures which have been subjected to other mechanical processes, such as creping, straining/activation by rolling with corrupted rolls or otherwise. Such mechanical process may be either alternative to the processes described hereinabove or in addition to such processes, i.e., sequentially either before or after such processes.

The absorbent core 24 may be any absorbent means which is capable of absorbing or retaining liquids (e.g., menses and/or urine). As shown in FIGS. 1 and 2, the absorbent core 24 has a body surface 24a, a garment facing surface 24b side edges, and end edges. The absorbent core 24 may be manufactured in a wide variety of sizes and shapes (e.g. rectangular, oval, hourglass, dogbone, asymmetric, etc.) and from a wide variety of liquid-absorbent materials commonly used in sanitary napkins and other absorbent articles such as communitive wood pulp which is generally referred to as airfelt. Examples of other suitable absorbent materials include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials; or any equivalent material or combination of materials, or mixtures of these. The configuration and construction of the absorbent core may also be varied (e.g., the absorbent core may have varying caliper zones (e.g. profiled so as to be thicker in the center), hydrophilic gradients, superabsorbent gradients or lower density or lower average basis weight acquisition zones; or may comprise one or more layers or structures). The total absorbent capacity of the absorbent core, should, however, be compatible with the design loading and the intended use of the absorbent article. Further, the size and absorbent capacity of the absorbent core may be varied to accommodate different uses such as incontinent pads, pantiliners, regular sanitary napkins, or overnight sanitary napkins.

Exemplary absorbent structures for use as the absorbent core in the present invention are described in U.S. Pat. No. 4,950,264 issued to Osborn on August 21, 1990; U.S. Pat. No. 4,610,678 issued to Weisman et al. on September 9, 1986; U.S. Pat. No. 4,834,735 issued to Alemany et al. on May 30, 1989; and European Patent Application No. 0 198 683, the Procter & Gamble Company, published October 22, 1986 in the name Duenk, et al. Each of these patents are incorporated herein by reference.

A preferred embodiment of the absorbent core 24 has a surface energy gradient similar to the surface energy gradient of the topsheet 22. The body facing surface 24a of the absorbent core and the portion of the absorbent core 24 immediately adjacent the body facing surface 24a preferably has a relatively low surface energy as compared to the garment facing surface 24b which has a relatively high surface energy. It is important to note that while there is a surface energy gradient within the absorbent core 24, the surface energy of the wearer-contacting or the body facing surface 24a of the absorbent core is preferably greater than the surface energy of the garment facing surface 25b of the acquisition layer 25. This relationship is preferred in order that fluid may be pulled or driven from the acquisition layer into the absorbent core. If the surface energy of the body facing surface 24a of the absorbent core were less than that of the garment facing surface 25b of the acquisition layer fluid in the acquisition layer 25 would be repelled by the absorbent core, thus rendering the absorbent core useless.

The backsheet 23 and the topsheet 22 are positioned adjacent the garment facing surface and the body facing surface respectively of the absorbent core 24 and are preferably joined thereto and to each other by attachment means (not shown) such as those well known in the art. For example, the backsheet 23 and/or the topsheet 22 may be secured to the absorbent core or to each other by a uniform continuous layer of adhesive, a patterned layer of adhesive or any array of separate lines, spirals or spots of adhesive. Adhesives which have been found to be satisfactory are manufactured by H.B. Fuller Company of St. Paul, Minnesota under the designation HL-1258 or H-2031. The attachment means will preferably comprise an open pattern network of filaments of adhesive as disclosed in U.S. Pat. No. 4,573,986 issued to Minetola et al. on March 4, 1986, and which is incorporated herein by reference. An exemplary attachment means of an open patterned network of filaments comprises several lines of adhesive filaments swirled into a spiral pattern such as illustrated by the apparatus and method shown in U.S. Pat. No. 3,911,173 issued to Sprague, Jr. on October 7, 1975; U.S. Pat. No. 4,785,996 issued to Zieker, et al. on November 22, 1978 and U.S. Pat. No. 4,842,666 issued to Werenicz on June 27, 1989. Each of these patents are incorporated herein by reference. Alternatively, the attachment means may comprise heat bonds, pressure bonds, ultrasonic bonds, dynamic mechanical bonds or any other suitable attachment means or combinations of these attachment means as are known in the art.

The backsheet 23 is impervious to liquids (e.g., menses and/or urine) and is preferably manufactured from a thin plastic film, although other flexible liquid impervious materials may also be used. As used herein, the term "flexible" refers to materials which are compliant and are more readily conformed to the general shape and contours of the human body. The backsheet 23 prevents the exudates absorbed and contained in the absorbent core from wetting articles which contact the sanitary napkin 20 such as pants, pajamas and undergarments. The backsheet 23 may thus comprise a woven or nonwoven material, polymeric films such as thermoplastic films of polyethylene or polypropylene, or composite materials such as a film-coated nonwoven material. Preferably, the backsheet of the polyethylene film having a thickness of from about 0.012 mm (0.5 mil) to about 0.051 mm (2.0 mil). Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance. Further, the backsheet 23 may permit vapors to escape from the absorbent core 24 (i.e., breathable) while still preventing exudates from passing through the backsheet 23.

In use, the sanitary napkin 20 can be held in place by any support means or attachment means (not shown) well-known for such purposes. Preferably, the sanitary napkin is placed in the user's undergarment or panty and secured thereto by a fastener such as an adhesive. The adhesive provides a means for securing the sanitary napkin in the crotch portion of the panty. Thus, a portion or all of the outer or garment facing surface 23b of the backsheet 23 is coated with adhesive. Any adhesive or glue used in the art for such purposes can be used for the adhesive herein, with pressure-sensitive adhesives being preferred. Suitable adhesives are Century A-305-IV manufactured by the Century Adhesives Corporation of Columbus, Ohio; and Instant Lock 34-2823 manufactured by the National Starch and Chemical Company of Bridgewater, NJ. Suitable adhesive fasteners are also described in U.S. Patent 4,917,697. Before the sanitary napkin is placed in use, the pressure-sensitive adhesive is typically covered with a removable release liner 37 in order to keep the adhesive from drying out or adhering to a surface other than the crotch portion of the panty prior to use. Suitable release liners are also described in the above-referenced U.S. Patent 4,917,697. Any commercially available release liners commonly used for such purposes can be utilized herein. Non-limiting examples of suitable release liners are BL30MG-A Silox E1/0 and BL30MG-A Silox 4P/O both of which are manufactured by the Akrosil Corporation of Menasha, WI. The sanitary napkin 20 of the present invention is used by removing the release liner and thereafter placing the sanitary napkin in a panty so that the adhesive contacts the panty. The adhesive maintains the sanitary napkin in its position within the panty during use.

In a preferred embodiment of the present invention, the sanitary napkin has two flaps 34 each of which are adjacent to and extend laterally from the side edge of the absorbent core. The flaps 34 are configured to drape over the edges of the wearer's panties in the crotch region so that the flaps are disposed between the edges of the wearer's panties and the thighs. The flaps serve at least two purposes. First, the flaps help serve to prevent soiling of the wearer's body and panties by menstrual fluid, preferably by forming a double wall barrier along the edges of the panty. Second, the flaps are preferably provided with attachment means on their garment surface so that the flaps can be folded back under the panty and attached to the garment facing side of the panty. In this way, the flaps serve to keep the sanitary napkin property positioned in the panty. The flaps can be constructed of various materials including materials similar to the topsheet, backsheet, tissue, or combination of these materials. Further, the flaps may be a separate element attached to the main body of the napkin or can comprise extensions of the topsheet and backsheet (i.e., unitary). A number of sanitary napkins having flaps suitable or adaptable for use with the sanitary napkins of the present invention are disclosed in U.S. 4,687,478 entitled "Shaped Sanitary Napkin With Flaps", which issued to Van Tilburg on August 18, 1987; U.S. 4,589,876 entitled "Sanitary Napkin", which issued to Van Tilburg on May 20, 1986; and U.S. 4,608,047, entitled "Sanitary Napkin Attachment Means", which issued to Mattingly on August 26, 1986. Each of these patents are incorporated herein by reference.

In a preferred embodiment of the present invention, an acquisition layer(s) 25 may be positioned between the topsheet 22 and the absorbent core 24. The acquisition layer 25 may serve several functions including improving wicking of exudates over and into the absorbent core. There are several reasons why the improved wicking of exudates is important, including providing a more even distribution of the exudates throughout the absorbent core and allowing the sanitary napkin 20 to be made relatively thin. (The wicking referred to herein may encompass the transportation of liquids in one, two or all directions (i.e, in the x-y plane and/or in the z-direction). The acquisition layer may be comprised of several different materials including nonwoven or woven webs of synthetic fibers including polyester, polypropylene, or polyethylene; natural fibers including cotton or cellulose; blends of such fibers; or any equivalent materials or combinations of materials. Examples of sanitary napkins having an acquisition layer and a topsheet are more fully described in U.S. 4,950,264 issued to Osborn and U.S. Patent Application Serial No. 07/810,774, "Absorbent Article Having Fused Layers", filed December 17, 1991 in the names of Cree, et al. Each of these references are incorporated herein by reference. In a preferred embodiment, the acquisition layer may be joined with the topsheet by any of the conventional means for joining webs together, most preferably by fusion bonds as is more fully described in the above-referenced Cree application.

In a preferred embodiment the acquisition layer 25 preferably has a surface energy gradient similar to that of the topsheet 22 and/or absorbent core 24. In a preferred embodiment, the first or wearer-facing surface 25a preferably has a relatively low surface energy as compared to the absorbent pad contacting surface 25b. Preferably, the surface energy of the first surface 25a of the acquisition layer 25 is preferably greater than the surface energy of the second surface of the topsheet 22. Furthermore, the second surface of the acquisition layer 25b has a relatively low surface energy compared to the surface energy of the body facing surface 24a of the absorbent core 24.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A fluid-pervious web (80) having a first surface (90) and a second surface (85) and exhibiting a multiplicity of apertures (81) said first (90) and said second surfaces being separated from one another by an intermediate portion (83) defined by the sidewalls of the apertures said web being coated on said first surface (90) characterized in that said first surface (90) of said web exhibits a surface energy less than said intermediate portion (83).

2. A fluid-pervious web according to claim 1, wherein said web is an apertured formed film.

3. The fluid-pervious web of Claim 2, further characterized in that said web is a microapertured formed film.

4. The fluid-pervious web of Claim 3, further characterized in that said web includes discrete volcano-like surface aberrations, said surface aberrations each including at least one microaperture.

5. The fluid-pervious web of Claim 4, further characterized in that said microapertured formed film includes a plurality of microapertures, and wherein at least a portion of said microapertures are at least partially obstructed by said coating.

6. The fluid-pervious web of any one of Claims 2 to 5, further characterized in that said apertured, formed film comprises a multilayer film having a first layer and a second layer, said second layer having a greater ductility than the ductility of said first layer, said second layer having a greater surface energy than the surface energy of said first layer.

7. A fluid-pervious web (80) according to claim 1, wherein said web is a coated nonwoven said first surface (90) of said web having a fluid impervious coating thereon and a plurality of apertures extending from said coating to said second surface of said web to render said web fluid pervious, characterized in that said coating exhibits a surface energy less than said intermediate portion, said intermediate portion preferably exhibiting a surface energy less than said second surface, said first surface of said web preferably including a plurality of zones exhibiting different surface energies.

8. An absorbent article including a topsheet, a backsheet secured to said topsheet, and an absorbent core positioned between said topsheet and said backsheet, said absorbent article preferably further including an acquisition layer positioned between said topsheet and said absorbent core, said absorbent article having a longitudinal axis, a transverse axis perpendicular to said longitudinal axis and a "Z" axis extending perpendicular to both said longitudinal axis and said transverse axis, said topsheet comprising a web according to claim 1.

9. The absorbent article of Claim 8, further characterized in that said topsheet is an apertured formed film.

10. The absorbent article of Claim 8, further characterized in that said topsheet is a coated, apertured, nonwoven web.

## Patentansprüche

1. Eine fluiddurchlässige Bahn (80), welche eine erste Oberfläche (90) und eine zweite Oberfläche (85) hat und eine Mehrzahl von Öffnungen (81) aufweist, wobei die genannte erste (90) und die genannte zweite Oberfläche (85) durch einen dazwischenliegenden Abschnitt (83) voneinander getrennt sind, welcher von den Seitenwandungen der Öffnungen begrenzt ist, wobei die genannte Bahn an der genannten ersten Oberfläche (90) beschichtet ist, dadurch gekennzeichnet, daß die genannte erste Oberfläche (90) der genannten Bahn eine Oberflächenenergie aufweist, welche geringer ist als die des genannten dazwischenliegenden Abschnitts (83).

2. Eine fluiddurchlässige Bahn nach Anspruch 1, bei welcher die genannte Bahn eine mit Öffnungen versehene geformte Folie ist.

3. Die fluiddurchlässige Bahn nach Anspruch 2, welche weiters dadurch gekennzeichnet ist, daß die genannte Bahn eine mit Mikroöffnungen versehene geformte Folie ist.

**4.** Die fluiddurchlässige Bahn nach Anspruch 3, welche weiters dadurch gekennzeichnet ist, daß die genannte Bahn diskrete vulkanähnliche Oberflächenaberrationen inkludiert, wobei die genannten Oberflächenaberrationen jeweils mindestens eine Mikroöffnung enthalten.

**5.** Die fluiddurchlässige Bahn nach Anspruch 4, welche weiters dadurch gekennzeichnet ist, daß die genannte mit Mikroöffnungen versehene geformte Folie eine Mehrzahl von Mikroöffnungen enthält und bei welcher mindestens ein Anteil der genannten Mikroöffnungen mindestens teilweise durch die genannte Beschichtung blockiert ist.

**6.** Die fluiddurchlässige Bahn nach einem der Ansprüche 2 bis 5, welche weiters dadurch gekennzeichnet ist, daß die genannte mit Öffnungen versehene geformte Folie eine mehrschichtige Folie aufweist, welche eine erste Schichte und eine zweite Schichte umfaßt, wobei die genannte zweite Schichte eine größere Duktilität als die Duktilität der genannten ersten Schichte umfaßt, die genannte zweite Schichte eine größere Oberflächenenergie als die Oberflächenenergie der genannten ersten Schichte aufweist.

**7.** Eine fluiddurchlässige Bahn (80) nach Anspruch 1, bei welcher die genannte Bahn ein beschichtetes Faservlies ist, die genannte erste Oberfläche (90) der genannten Bahn eine fluidundurchlässige Beschichtung daran und eine Mehrzahl von Öffnungen, welche sich von der genannten Beschichtung zur genannten zweiten Oberfläche der genannten Bahn erstrecken, um die genannte Bahn fluiddurchlässig zu machen, aufweist, dadurch gekennzeichnet, daß die genannte Beschichtung eine Oberflächenenergie, welche geringer ist als die des genannten dazwischenliegenden Abschnitts, aufweist, wobei der genannte dazwischenliegende Abschnitt vorzugsweise eine Oberflächenenergie geringer als die der genannten zweiten Oberfläche aufweist, die genannte erste Oberfläche der genannten Bahn vorzugsweise eine Mehrzahl von Zonen enthält, welche unterschiedliche Oberflächenenergien aufweisen.

**8.** Ein absorbierender Artikel, welcher ein Deckblatt, ein am genannten Deckblatt fixiertes Rückenblatt und einen zwischen dem genannten Deckblatt und dem genannten Rückenblatt positionierten absorbierenden Kern enthält, wobei der absorbierende Artikel vorzugsweise weiters eine Erfassungsschichte enthält, welche zwischen dem genannten Deckblatt und dem genannten absorbierenden Kern positioniert ist, wobei der genannte absorbierende Artikel eine Längsachse, eine zur genannten Längsachse lotrechte Querachse und eine „Z"-Achse aufweist, welche sich lotrecht zu sowohl der genannten Längsachse als auch der genannten Querachse erstreckt, wobei das genannte Deckblatt eine Bahn nach Anspruch 1 aufweist.

**9.** Der absorbierende Artikel nach Anspruch 8, welcher weiters dadurch gekennzeichnet ist, daß das genannte Deckblatt eine mit Öffnungen versehene geformte Folie ist.

**10.** Der absorbierende Artikel nach Anspruch 8, welcher weiters dadurch gekennzeichnet ist, daß das genannte Deckblatt eine beschichtete, mit Öffnungen versehene Faservliesbahn ist.

**Revendications**

**1.** Nappe (80) perméable aux fluides, ayant une première surface (90) et une deuxième surface (85) et présentant une pluralité de perforations (81), ladite première surface (90) et ladite deuxième surface (85) étant séparées l'une de l'autre par une partie intermédiaire (83) définie par les parois latérales des perforations, ladite nappe étant pourvue d'un revêtement sur ladite première surface (90), caractérisée en ce que ladite première surface (90) de ladite nappe présente une énergie superficielle inférieure à celle de ladite partie intermédiaire (83).

**2.** Nappe perméable aux fluides selon la revendication 1, dans laquelle ladite nappe est un film formé, perforé.

**3.** Nappe perméable aux fluides selon la revendication 2, caractérisée, en outre, en ce que ladite nappe est un film formé, microperforé.

**4.** Nappe perméable aux fluides selon la revendication 3, caractérisée, en outre, en ce que ladite nappe comprend des aberrations de surface discrètes, en forme de volcans, lesdites aberrations de surface comprenant chacune au moins une microperforation.

**5.** Nappe perméable aux fluides selon la revendication 4, caractérisée, en outre, en ce que ledit film formé, microperforé, comprend une pluralité de microperforations, et dans laquelle au moins une partie desdites microperforations est au moins partiellement obturée par ledit revêtement.

**6.** Nappe perméable aux fluides selon l'une quelconque des revendications 2 à 5, caractérisée, en outre, en ce que ledit film formé, perforé, comprend un film multicouche ayant une première couche et une deuxième couche, ladite deuxième couche ayant une ductilité supérieure à celle de ladite première couche, ladite deuxième couche ayant une énergie superficielle supérieure à celle de ladite première couche.

7. Nappe perméable aux fluides (80) selon la revendication 1, dans laquelle ladite nappe est un non tissé pourvu d'un revêtement, ladite première surface (90) de ladite nappe ayant un revêtement imperméable aux fluides déposé sur elle et une pluralité de perforations qui s'étendent depuis ledit revêtement jusqu'à ladite deuxième surface de ladite nappe pour rendre ladite nappe perméable aux fluides, caractérisée en ce que ledit revêtement présente une énergie superficielle inférieure à celle de la partie intermédiaire, ladite partie intermédiaire présentant, de préférence, une énergie superficielle inférieure à celle de ladite deuxième surface, ladite première surface de ladite nappe comprenant, de préférence, une pluralité de zones présentant différentes énergies superficielles.

8. Article absorbant comprenant une feuille de dessus, une feuille de fond fixée à ladite feuille de dessus, et une âme absorbante positionnée entre ladite feuille de dessus et ladite feuille de fond, ledit article absorbant comprenant, de préférence, en outre une couche de recueil positionnée entre ladite feuille de dessus et ladite âme absorbante, ledit article absorbant ayant un axe longitudinal, un axe transversal perpendiculaire audit axe longitudinal, et un axe "Z" s'étendant perpendiculairement audit axe longitudinal et audit axe transversal, ladite feuille de dessus étant constituée d'une nappe selon la revendication 1.

9. Article absorbant selon la revendication 8, caractérisée, en outre, en ce que ladite feuille de dessus est un film formé, perforé.

10. Article absorbant selon la revendication 8, caractérisée, en outre, en ce que ladite feuille de dessus est une nappe non tissée, munie de perforations et d'un revêtement.

Fig. 1

Fig. 2

Fig. 3

(PRIOR ART)

Fig. 4

Fig. 5

Fig. 6

Fig. 7

Fig. 8

Fig. 9

Fig. 10

Fig. 11

EP 0 767 646 B1

Fig. 12

Fig. 13

Fig. 14

Fig. 15

Fig. 16

Fig. 17